(19) Europäisches Patentamt
European Patent Office
Office européen des brevets

(11) EP 1 092 715 B1

(12) **FASCICULE DE BREVET EUROPEEN**

(45) Date de publication et mention de la délivrance du brevet:
**23.03.2005 Bulletin 2005/12**

(21) Numéro de dépôt: **00402799.1**

(22) Date de dépôt: **11.10.2000**

(51) Int Cl.[7]: **C07D 405/14**, A61K 31/505, A61K 31/53, A61K 31/54, A61K 31/40, C07D 403/14, C07D 471/04, C07D 513/04, C07D 417/14, C07D 487/04, A61P 1/08, A61P 25/00, A61P 25/24, A61P 43/00 // (C07D471/04, 251:00, 221:00), (C07D487/04, 209:00, 209:00)

(54) **Composés cyano-indoles inhibiteurs de recapture de sérotonine, leur procédé de préparation et les compositions pharmaceutiques qui les contiennent**

Cyanoindol-Derivate als Inhibitoren der Wiederaufnahme von Serotonin, Verfahren zu ihrer Herstellung und sie enthaltende pharmazeutische Zubereitungen

Cyano-indole derivatives as inhibitors of serotonin reuptake, method for their preparation and pharmaceutical compositions containing the same

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **12.10.1999 FR 9912668**

(43) Date de publication de la demande:
**18.04.2001 Bulletin 2001/16**

(73) Titulaire: **LES LABORATOIRES SERVIER**
**92200 Neuilly sur Seine (FR)**

(72) Inventeurs:
- **Lavielle, Gilbert**
  **78170 La Celle Saint Cloud (FR)**
- **Muller, Olivier**
  **95300 Ennery (FR)**
- **Millan, Mark**
  **78230 Le Pecq (FR)**
- **Cussac, Didier**
  **78400 Chatou (FR)**
- **Dekeyne, Anne**
  **78470 Saint Remy les Chevreuses (FR)**

(56) Documents cités:
**EP-A- 0 464 558**
**EP-A- 0 610 134**
**EP-A- 0 814 084**
**WO-A-97/17343**
**WO-A-98/27089**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 092 715 B1

## Description

La présente invention concerne de nouveaux composés cyano-indoles inhibiteurs de recapture de sérotonine, leur procédé de préparation et les compositions pharmaceutiques qui les contiennent.

Des composés caractérisés par l'association d'un noyau indole et d'un noyau 2,3-dihydro-1,4-benzodioxine ont été décrits pour leurs propriétés inhibitrices de recapture de la sérotonine (WO 9717343). De même des indoles substitués sur le noyau aromatique sont revendiqués dans les demandes EP 610134 et EP 814084 pour leur action respectivement comme agonistes $5HT_{1\text{-like}}$ et au niveau des sites de recapture de la sérotonine. D'autre composés possédant des propriétés voisines sont revendiqués dans la demande WO 9633710 et possèdent un squelette benzopyrane.

Les inhibiteurs de recapture de la sérotonine constituent un groupe hétérogène d'agents thérapeutiques. Ils trouvent leur utilisation dans le traitement de pathologies associées à un déficit en sérotonine au niveau des synapses des neurones centraux. L'inhibition de la recapture de la sérotonine par liaison avec des transporteurs ou des récepteurs présynaptiques est un moyen de restaurer la transmission nerveuse.

L'utilisation de composés possédant ces propriétés inhibitrices peut constituer une alternative à l'emploi d'antidépresseurs tricycliques ou d'inhibiteurs de monoamine oxydase, dans le traitement de la dépression et des troubles associés (Annals of Pharmacotherapy, 1994, 28, 1359), des attaques de panique et des troubles obsessionnels compulsifs (Human Psychopharmacology, 1995, 10, 5199). L'efficacité de composés possédant de telles propriétés pharmacologiques (Journal of Psychopharmacology, 1994, 8, 238) se trouve renforcée par leur plus grande tolérabilité (International Clinical Psychopharmacology, 1995, 9 suppl. 4, 33) et leur plus grande sécurité d'utilisation (Annals of Pharmacology, ref. citée).

Les composés de la présente invention se caractérisent par un noyau indole substitué sur la partie benzénique par un groupement cyano, et en position 3 par un groupement 3-pyrrolylalkyle N-substitué. Cette structure originale leur confère un puissant caractère inhibiteur de recapture de la sérotonine. Ils seront donc utiles en thérapeutique pour le traitement de la dépression, des attaques de panique, des troubles obsessionnels compulsifs, des phobies, des troubles impulsifs de l'abus de drogue, de la boulimie nerveuse et de l'anxiété.

La présente invention concerne les composés de formule (I) :

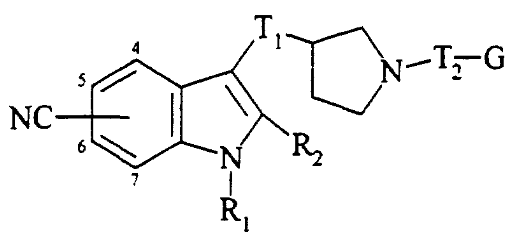

(I)

dans laquelle :

- $R_1$ et $R_2$ représentent indépendamment un atome d'hydrogène ou un groupement alkyle ($C_1$-$C_6$) linéaire ou ramifié,
- $T_1$ et $T_2$ représentent indépendamment un groupement alkylène ($C_1$-$C_6$) linéaire ou ramifié,
- G représente un groupement hétérocyclique de formule (α) ou (β) :

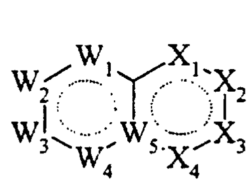

(α)

(ou)

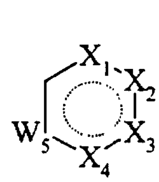

(β)

dans lesquelles :
$W_1$ à $W_5$ et $X_1$ à $X_4$ sont choisis de façon à former un groupement chimiquement stable, et sont définis de la manière suivante :

- $W_1$, $W_2$ et $W_3$ représentent indépendamment un atome d'azote, un groupement $CR_5$, NR4 ou CO,
- $W_4$ représente un atome d'azote, un groupement $CR_3$, $NR_4$ ou CO,
- $W_5$ représente un atome de carbone ou un atome d'azote,
- $X_1$ représente une liaison, un atome d'azote, ou un groupement $CR_3$ ou $NR_4$,
- $X_2$ à $X_4$ représentent indépendamment un groupement $CR_3$, $NR_4$, CO, $SR_4$ ou $SO_2$ ou bien un atome d'oxygène, de soufre ou d'azote,
- $R_3$ représente un atome d'hydrogène, d'halogène, un groupement alkyle ($C_1$-$C_6$) linéaire ou ramifié, hydroxy, perhalogénoalkyle ($C_1$-$C_6$) linéaire ou ramifié, nitro, ou amino (éventuellement substitué par un ou deux groupements alkyle ($C_1$-$C_6$) linéaire ou ramifié, ou benzyle),
- $R_4$ représente un atome d'hydrogène ou un groupement alkyle ($C_1$-$C_6$) linéaire ou ramifié, aryle éventuellement substitué, arylalkyle éventuellement substitué,
- $R_5$ représente un groupement $R_3$ ou bien deux groupements $R_5$ adjacents forment avec les atomes de carbone qui les portent un groupement mono ou bicyclique saturé, partiellement insaturé ou insaturé, contenant éventuellement de 1 à 5 hétéroatomes choisis parmi azote, oxygène et soufre, ledit groupement étant éventuellement substitué par un ou plusieurs groupements $R_3$ ou oxo,

étant entendu que dans les formules (α) et (β) au moins un hétéroatome est présent, que les pointillés signifient que ces groupements peuvent comporter une insaturation ou plusieurs insaturations conjuguées ou non, et que en cas d'absence d'insaturation les valences restantes sont occupées par des atomes d'hydrogène, les groupements (α) et (β) étant reliés à $T_2$ par l'un quelconque de leur sommet,
leurs énantiomères, diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...
Parmi les bases pharmaceutiquement acceptables, on peut citer à titre non limitatif l'hydroxyde de sodium, l'hydroxyde de potassium, la triéthylamine, la tertbutylamine, etc...
Par groupement aryle on entend un groupement phényle ou naphtyle.

L'expression éventuellement substituée affectée aux groupements aryle ou arylalkyle signifie que ces groupements sont éventuellement substitués par un ou plusieurs groupes choisis parmi un atome d'halogène, un groupement alkyle ($C_1$-$C_6$) linéaire ou ramifié, hydroxy, perhalogénoalkyle ($C_1$-$C_6$) linéaire ou ramifié, nitro, ou amino (éventuellement substitué par un ou deux groupements alkyle ($C_1$-$C_6$) linéaire ou ramifié, ou benzyle),

De façon avantageuse, l'invention concerne les composés de formule (I) pour lesquels le groupement cyano est rattaché en position 5 du noyau indole.

Un autre aspect avantageux de l'invention concerne les composés de formule (I) pour lesquels le groupement cyano est rattaché en position 6 du noyau indole.

De façon préférentielle dans les composés de formule (I), $R_1$ et $R_2$ représentent chacun un atome d'hydrogène.

Des composés préférés de l'invention sont ceux pour lesquels $T_1$ représente un groupement méthylène.

Le groupement aryle préféré de l'invention est le groupement phényle.
Dans les groupements G préférés de l'invention, $R_3$, $R_4$ et $R_5$ sont avantageusement choisis parmi les groupements alkyle ($C_1$-$C_6$) linéaires ou ramifiés et les atomes d'hydrogène et d'halogène.

Parmi les groupements G préférés de l'invention on peut citer de façon non limitative les groupements :
2-furyle ; 2,4-dioxo-1,4-dihydro-3(2*H*)-quinazolinyl ; 3-oxo-[1,2,4]triazolo[4,3-a] pyridin-2(3*H*)-yl ; 1-oxo-2(1*H*)-phtalazinyl ; 7-méthyl-5-oxo-5*H*-[1,3]thiazolo[3,2-a] pyrimidin-6-yl ; 6-chloro-2-oxo-2,3-dihydro-1*H*-indol-5-yl ; 2-oxo-2,3-dihydro-1*H*-indol-5-yl ; 2-oxo-1,2,3,4-tétrahydro-6-quinolinyl ; 3-benzyl-5-méthyl-2,6-dioxo-3,6-dihydro-1(2*H*)-pyrimidinyl ; 1,3-dioxo-1,3-dihydro-2*H*-isoindol-2-yl ; 1,1,3-trioxo-1,3-dihydro-2*H*-1,2-benzisothiazol-2-yl ; 1,3-dioxo-3,6-dihydropyrrolo[3,4-c]carbazol-2 (1*H*)-yl ; 1,3-dioxo-1,3-dihydro-2*H*-benzo[c]isoindol-2-yl ; 3,5-diméthyl-2,6-dioxo-3,6-dihydro-1(2*H*)-pyrimidinyl.

Un aspect particulièrement avantageux de l'invention concerne les composés de formule (I) pou lesquels $R_1$ et $R_2$ représentent chacun un atome d'hydrogène, $T_1$ représente un groupement méthylène, $T_2$ représente un groupement alkylène (par exemple méthylène ou éthylène) et G est choisi parmi les groupements 2-furyle ; 2,4-dioxo-1,4-dihydro-3(2*H*)-quinazolinyl ; 3-oxo-[1,2,4]triazolo[4,3-a] pyridin-2(3*H*)-yl ; 1-oxo-2(1*H*)-phtalazinyl ; 7-méthyl-5-oxo-5*H*-[1,3]thiazolo[3,2-a]pyrimidin-6-yl ; 6-chloro-2-oxo-2,3-dihydro-1*H*-indol-5-yl ; 2-oxo-2,3-dihydro-1*H*-indol-5-yl ; 2-oxo-1,2,3,4-tétrahydro-6-quinolinyl ; 3-benzyl-5-méthyl-2,6-dioxo-3,6-dihydro-1(2*H*)-pyrimidinyl ; 1,3-dioxo-1,3-dihydro-2*H*-isoindol-2-yl ; 1,1,3-trioxo-1,3-dihydro-2*H*-1,2-benzisothiazol-2-yl ; 1,3-dioxo-3,6-dihydropyrrolo[3,4-c]carbazol-2(1*H*)-yl ; 1,3-dioxo-1,3-dihydro-2*H*-benzo[c]isoindol-2-yl ; 3,5-diméthyl-2,6-dioxo-3,6-dihydro-1(2*H*)-pyrimidinyl

Parmi ces composés, on préfèrera ceux pour lesquels le groupement cyano est rattaché en position 5 ou 6 du noyau indole, tout particulièrement ceux pour lesquels le groupement cyano est rattaché en position 5.

Parmi les composés préférés de l'invention on peut citer tout particulièrement :

- 3-({1-[2-(7-Méthyl-5-oxo-5*H*)-[1,3]thiazolo[3,2-a]pyrimidin-6-yl)éthyl]-3-pyrrolidinyl}méthyl)-1*H*-indole-5-carbonitrile, chlorhydrate
- 3-({1-[2-(6-Chloro-2-oxo-2,3-dihydro-1*H*-indol-5-yl)éthyl]-3-pyrrolidinyl}méthyl)-1*H*-indole-5-carbonitrile, chlorhydrate.

L'invention s'étend également au procédé de préparation des composés de formule (I), caractérisé en ce que l'on utilise comme produit de départ un composé de formule (II) :

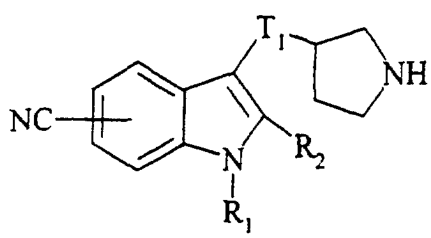

(II)

dans laquelle $R_1$, $R_2$ et $T_1$ sont tels que définis dans la formule (I), qui est soumis :

- soit à l'action, en milieu réducteur d'un aldéhyde de formule (III) :

$$OHC\text{-}T'_2\text{-}G \qquad (III)$$

dans laquelle G est tel que défini dans la formule (I), et $T'_2$ représente une liaison ou un groupement alkylène ($C_1$-$C_5$) linéaire ou ramifié,

- soit à l'action, en milieu basique d'un composé halogéné de formule (IV) :

$$Hal\text{-}T_2\text{-}G \qquad (IV)$$

dans laquelle $T_2$ est tel que défini dans la formule (I) et Hal représente un atome d'halogène,

pour conduire à un composé de formule (I),
ou bien qui est soumis à l'action, en milieu basique d'un dérivé de formule (V) :

$$Hal\text{-}T_2\text{-}NH\text{-}P' \qquad (V)$$

dans laquelle $T_2$ est tel que défini dans la formule (I), Hal représente un atome d'halogène, et P' un groupement protecteur de l'amine,
pour conduire à un composé de formule (VI),

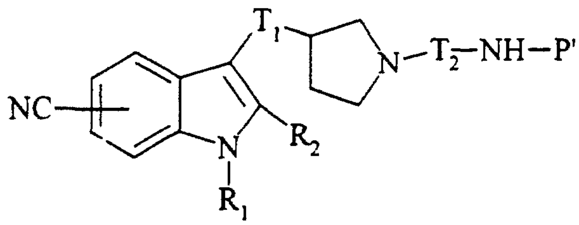

(VI)

dans laquelle $R_1$, $R_2$, $T_1$ $T_2$, P' sont tels que définis précédemment,
composé (VI) qui, après déprotection de l'amine primaire, est condensé sur un groupement cyclique, précurseur du groupement G défini dans la formule (I), pour conduire au composé de formule (I/a):

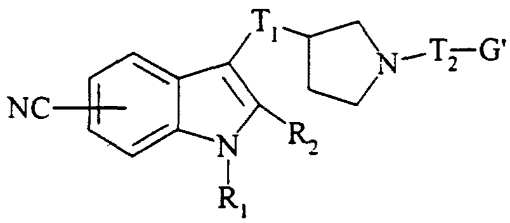

(I/a)

cas particulier des composés de formule (I) pour lequel $R_1$, $R_2$, $T_1$ $T_2$ sont tels que définis précédemment, et G' représente un groupement tel que défini pour G dans la formule (I), étant entendu que G' est rattaché à T2 par un atome d'azote,
composés de formule (I/a) et (I),

- qui peuvent être, le cas échéant, purifiés selon une technique classique de purification,
- dont on sépare, le cas échéant, les isomères selon une technique classique de séparation,
- que l'on transforme, si on le souhaite, en leurs sels d'addition à un acide ou à une base pharmaceutiquement acceptable.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptable.
Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...
La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.
Les exemples suivants illustrent l'invention et ne la limitent en aucune façon.
Les produits de départ utilisés sont des produits connus ou préparés selon des modes opératoires connus.

**PREPARATION A : 3-[(3-Pyrrolidinyl)méthyl]-1*H*-indole-5-carbonitrile**

L'hydrogénolyse de 0,32 mol (96 g) de 3-(1-benzyl-3-pyrrolidinylmethyl)-1*H*-indole-5-carbonitrile (décrit dans la demande EP 610134) est réalisée dans 1.7 litre d'éthanol en présence de 9.6 g de charbon à 20 % d'hydroxyde de palladium humide (catalyseur de Pearlman) et 0,32 mol d'acide chlorhydrique dans l'éthanol à 70°C et à pression atmosphérique. Après, absorption de la théorie en hydrogène, le catalyseur est filtré puis le filtrat concentré. La base est relarguée par de la soude 1 N et extraite à l'aide de dichlorométhane, pour conduire au produit attendu.

**PREPARATION B : 2-Bromoéthylcarbamate de tert-butyle**

Sous atmosphère inerte, à température ambiante, on prépare une solution de 62 mmol (10 g) de N-(tertbutoxycarbonyl)éthanolamine, dans 100 ml d'acétonitrile à laquelle 74,5 mmol (24.7 g) de tétrabromométhane sont ajoutées. Puis 68,2 mmol (17.9 g) de triphénylphosphine en solution dans 150 ml d'acétonitrile sont additionnées goutte

à goutte. Le milieu réactionnel est agité à température ambiante pendant 30 minutes, puis concentré. Le résidu est repris dans l'éther isopropylique, et après filtration, le filtrat est concentré pour obtenir le produit du titre.

**EXEMPLE 1 : -3-{[1-(2-Furylméthyl)-3-pyrrolidinyl]méthyl}-1*H*-indole-5-carbonitrile, oxalate**

Sous atmosphère inerte, on dissout à température ambiante 47,3 mmol (10 g) de 3-[3-pyrrolidinylméthyl]-1*H*-indole-5-carbonitrile (décrit dans la préparation A), dans 650 ml de 1,2-dichloroéthane, puis 47,3 mmol (4.26 g) de furaldéhyde sont ajoutées. L'agitation est maintenue 10 minutes et 66,2 mmol (13.79 g) de triacétoxyborohydrure de sodium sont ajoutées. Après 30 minutes à température ambiante, on additionne 350 ml d'une solution saturée d'hydrogénocarbonate de sodium. La phase organique est décantée, lavée à l'eau, séchée sur sulfate de magnésium.
La purification est effectuée par chromatographie sur silice en utilisant comme éluant un mélange dichlorométhane/méthanol/ammoniaque 98/2/0,2. Le composé obtenu est salifié par un équivalent d'acide oxalique dans l'éthanol.

*Point de fusion :* 120-122°C

| *Microanalyse élémentaire :* | | | |
|---|---|---|---|
| | C | H | N |
| % calculé<br>% trouvé | 63.79<br>63.54 | 5.35<br>5.58 | 10.63<br>10.37 |

**EXEMPLE 2 : 3-[(1-{2-[2,4-Dioxo-1,4-dihydro-3(2*H*)-quinazolinyl]éthyl}-3-pyrrolidinyl)méthyl]-1*H*-indole-5-carbonitrile, chlorhydrate**

Sous atmosphère inerte, on dissout à température ambiante 47,3 mmol (10 g) de 3-[3-pyrrolidinylméthyl]-1*H*-indole-5-carbonitrile, 47,3 mmol (9.97g) de 3-(2-chloroéthyl)-2,4-(1*H*,3*H*)-quinazolinedione, 47,3 mmol (3.73 g) d'hydrogénocarbonate de sodium dans 100 ml de diméthylformamide, et on agite à 100°C pendant 6 heures. Le milieu réactionnel est concentré, le résidu est repris par du dichlorométhane. La phase organique est lavée à l'eau, puis par une solution saturée de chlorure de sodium, et séchée sur sulfate de magnésium. La purification est effectuée par chromatographie sur silice en utilisant comme éluant un mélange dichlorométhane/méthanol/ammoniaque 95/5/0.5. Le composé obtenu est salifié par un équivalent d'acide chlorhydrique dans l'éthanol.

*Point de fusion :* 152-154°C

| *Microanalyse élémentaire :* | | | | |
|---|---|---|---|---|
| | C | H | N | Cl |
| % calculé<br>% trouvé | 64.07<br>63.81 | 5.38<br>5.34 | 15.57<br>15.20 | 7.88<br>7.64 |

**EXEMPLE 3 : 3-[(1-{2-[3-Oxo-[1,2,4]triazolo[4,3-*a*]pyridin-2(3*H*)-yl]éthyl}-3-pyrrolidinyl)méthyl]-1*H*-indole-5-carbonitrile, chlorhydrate**

Le produit attendu est obtenu selon le procédé décrit dans l'exemple 2, en remplaçant la 3-(2-chloroéthyl)-2,4-(1*H*,3*H*)-quinazolinedione par la 2-(2-chloroéthyl)-[1,2,4]triazolo [4,3-*a*]pyridin-3(2*H*)-one.

<u>Point de fusion :</u> 140°C

| *Microanalyse élémentaire :* | | | | |
|---|---|---|---|---|
| | C | H | N | Cl |
| % calculé | 62.48 | 5.48 | 19.87 | 8.38 |
| % trouvé | 62.48 | 5.68 | 19.54 | 8.48 |

**<u>EXEMPLE 4 :</u> 3-[(1-{2-[1-Oxo-2(1*H*)-phthalazinyl]éthyl}-3-pyrrolidinyl)méthyl]-1*H*-indole-5-carbonitrile, chlorhydrate**

Le produit attendu est obtenu selon le procédé décrit dans l'exemple 2, en remplaçant la 3-(2-chloroéthyl)-2,4-(1*H*,3*H*)-quinazolinedione par la 2-(2-chloroéthyl)-1(2*H*)-phthalazinone.

<u>Point de fusion :</u> 125-127°C

| *Microanalyse élémentaire :* | | | | |
|---|---|---|---|---|
| | C | H | N | Cl |
| % calculé | 66.43 | 5.57 | 16.14 | 8.17 |
| % trouvé | 66.74 | 5.63 | 15.79 | 8.27 |

**<u>EXEMPLE 5 :</u> 3-({1-[2-(7-Méthyl-5-oxo-(5*H*)-[1,3]thiazolo[3,2-*a*]pyrimidin-6-yl) éthyl]-3-pyrrolidinyl}méthyl)-1*H*-indole-5-carbonitrile, chlorhydrate**

Le produit attendu est obtenu selon le procédé décrit dans l'exemple 2, en remplaçant la 3-(2-chloroéthyl)-2,4-(1*H*,3*H*)-quinazolinedione par la 6-(2-chloroéthyl)-7-méthyl-5*H*-[1,3]thiazolo[3,2-*a*]pyrimidin-5-one.

<u>Point de fusion :</u> 234-236°C

| *Microanalyse élémentaire :* | | | | | |
|---|---|---|---|---|---|
| | C | H | N | S | Cl |
| % calculé | 60.85 | 5.33 | 15.43 | 7.06 | 7.81 |
| % trouvé | 59.83 | 5.38 | 14.69 | 6.90 | 7.69 |

**<u>EXEMPLE 6 :</u> 3-({1-[2-(6-Chloro-2-oxo-2,3-dihydro-1*H*-indol-5-yl)éthyl]-3-pyrrolidinyl}méthyl)-1*H*-indole-5-carbonitrile, chlorhydrate**

Le produit attendu est obtenu selon le procédé décrit dans l'exemple 2, en remplaçant la 3-(2-chloroéthyl)-2,4-(1*H*,3*H*)-quinazolinedione par la 6-chloro-5-(2-chloroéthyl)-1,3-dihydro-2*H*-indol-2-one.

<u>Point de fusion :</u> 115-117°C

| *Microanalyse élémentaire :* | | | | |
|---|---|---|---|---|
| | C | H | N | Cl |
| % calculé | 63.30 | 5.31 | 12.30 | 15.57 |

(suite)

| *Microanalyse élémentaire :* | | | | |
|---|---|---|---|---|
| | C | H | N | Cl |
| % trouvé | 63.57 | 5.42 | 12.06 | 15.27 |

**EXEMPLE 7 : 3-({1-[2-(2-Oxo-2,3-dihydro-1*H*-indol-5-yl)éthyl]-3-pyrrolidinyl} méthyl)-1*H*-indole-5-carbonitrile, chlorhydrate**

Le produit attendu est obtenu selon le procédé décrit dans l'exemple 2, en remplaçant la 3-(2-chloroéthyl)-2,4-(1*H*,3*H*)-quinazolinedione par la 5-(2-chloroéthyl)-1,3-dihydro-2*H*-indol-2-one.

*Point de fusion :* 118-120°C

| *Microanalyse élémentaire :* | | | | |
|---|---|---|---|---|
| . | C | H | N | Cl |
| % calculé<br>% trouvé | 68.48<br>68.85 | 5.99<br>6.30 | 13.31<br>12.81 | 8.42<br>8.50 |

**EXEMPLE 8 : 3-({1-[2-(2-Oxo-1,2,3,4-tetrahydro-6-quinolinyl)éthyl]-3-pyrrolidinyl}méthyl)-1*H*-indole-5-carbonitrile, chlorhydrate**

Le produit attendu est obtenu selon le procédé décrit dans l'exemple 2, en remplaçant la 3-(2-chloroéthyl)-2,4-(1*H*,3*H*)-quinazolinedione par la 6-(2-bromoéthyl)-3,4-dihydro-2(1*H*)-quinolinone.

*Point de fusion :* 110°C (déc.)

| *Microanalyse élémentaire :* | | | | |
|---|---|---|---|---|
| | C | H | N | Cl |
| % calculé<br>% trouvé | 69.03<br>69.10 | 6.26<br>6.40 | 12.88<br>12,31 | 8.15<br>8.19 |

**EXEMPLE 9 : 3-[(1-{2-[3-Benzyl-5-méthyl-2,6-dioxo-3,6-dihydro-1(2*H*)-pyrimidinyl]éthyl}-3-pyrrolidinyl)méthyl]-1*H*-indole-5-carbonitrile**

Le produit attendu est obtenu selon le procédé décrit dans l'exemple 2, en remplaçant la 3-(2-chloroéthyl)-2,4-(1*H*,3*H*)-quinazolinedione par la 1-benzyl-3-(2-chloroéthyl)-5-méthyl-2,4-(1*H*,3*H*)-pyrimidinedione.

*Point de fusion :* 130-132°C

| *Microanalyse élémentaire :* | | | | |
|---|---|---|---|---|
| | C | H | N | Cl |
| % calculé<br>% trouvé | 66.72<br>66.49 | 6.00<br>6.12 | 13.89<br>13.38 | 7.03<br>6.99 |

**EXEMPLE 10 : 3-({1-[2-(1,3-Dioxo-1,3-dihydro-2*H*-isoindol-2-yl)éthyl]-3-pyrrolidinyl}méthyl)-1*H*-indole-5-carbonitrile, chlorhydrate**

*Etape 1 : 2-{3-[(5-Cyano-1H-indol-3-yl)méthyl]-1-pyrrolidinyl}éthylcarbamate de tert-butyle*

Sous atmosphère inerte, on mélange 47,3 mmol (10 g) de 3-[(3-pyrrolidinyl)méthyl]-1*H*-indole-5-carbonitrile, 47,3 mmol (9.95 g) du produit décrit dans la préparation B, et 1,55 ml de 2-butanone, puis 47,3 mmol (4.45g) d'hydrogénocarbonate de potassium sont ajoutées. Le milieu est agité à 80°C pendant 2 heures. Après hydrolyse et décantation, la phase organique est lavée avec une solution de chlorure de sodium saturée, séchée sur sulfate de magnésium, filtrée, concentrée. La purification est effectué par chromatographie sur silice, en utilisant comme éluant un mélange dichlorométhane/méthanol/ammoniaque 97/3/0.3 pour conduire au produit attendu.

*Etape 2 : 3-{[1-(2-Aminoéthyl)-3-pyrrolidinyl]méthyl}-1H-indole-5-carbonitrile*

A température ambiante on dissout 44,4 mmol (5 g) du produit décrit à l'étape précédente, dans un mélange de 150 ml d'acide chlorhydrique 3N et 100 ml d'acétate d'éthyle. Le milieu est agité à cette température 30 minutes. Après décantation des deux phases, la phase acide est concentrée. Le résidu est repris dans la soude 0.1 N puis extrait au dichlorométhane. La phase organique est lavée à l'eau, puis par une solution de chlorure de sodium saturée, séchée sur sulfate de magnésium, filtrée, et concentrée, pour obtenir le produit du titre.

*Etape 3: 3-({1-[2-(1,3-Dioxo-1,3-dihydro-2H-isoindol-2-yl)éthyl]-3-pyrrolidinyl}méthyl)-1H-indole-5-carbonitrile, chlorhydrate*

On mélange successivement 11,2 mmol (3 g) du produit décrit à l'étape précédente, 12,3 mmol (1.82 g) d'anhydride phtalique, puis 28 ml d'acide acétique glacial. La réaction est chauffée au reflux pendant 1h, puis l'acide acétique évaporé. Le résidu est repris par de la soude 1 N et extrait au dichlorométhane. La phase organique est lavée à l'eau, puis par une solution de chlorure de sodium saturée, séchée sur sulfate de magnésium, filtrée, et concentrée. La purification est effectuée par chromatographie sur silice, en utilisant comme éluant un mélange dichlorométhane/méthanol/ammoniaque 97/3/0.3. Le produit obtenu est salifié par un équivalent d'acide chlorhydrique dans l'éthanol.

*Point de fusion :* 140-142°C

| *Microanalyse élémentaire* : | | | | |
|---|---|---|---|---|
| | C | H | N | Cl |
| % calculé | 66.28 | 5.33 | 12.88 | 8.15 |
| % trouvé | 65.68 | 5.42 | 12.30 | 8.32 |

**EXEMPLE 11 : 3-({1-[2-(1,1,3-Trioxo-1,3-dihydro-2*H*-1,2-benzisothiazol-2-yl)éthyl] -3-pyrrolidinyl}méthyl)-1*H*-indole-5-carbonitrile, chlorhydrate**

Le produit attendu est obtenu selon le procédé décrit dans l'exemple 10, en remplaçant l'anhydride phtalique par le 2-chlorosulfonylbenzoate de méthyle.

**EXEMPLE 12 : 3-({1-[2-(1,3-Dioxo-1,3-dihydro-2*H*-benzo[*e*]isoindol-2-yl)éthyl] -3-pyrrolidinyl}méthyl)-1*H*-indole-5-carbonitrile, chlorhydrate**

Le produit attendu est obtenu selon le procédé décrit dans l'exemple 10, en remplaçant l'anhydride phtalique par le naphtho[1,2-*c*]furan-1,3-dione.

*Point de fusion :* 112-116°C

| *Microanalyse élémentaire :* | | | | |
|---|---|---|---|---|
| | C | H | N | Cl |
| % calculé | 69.34 | 5.20 | 11.55 | 7.31 |
| % trouvé | 68.58 | 5.49 | 11.10 | 6.90 |

**EXEMPLE 13 : 3-[(1-{2-[6-Méthyl-1,3-dioxo-3,6-dihydropyrrolo[3,4-*c*]carbazol-2(1*H*)-yl]éthyl}-3-pyrrolidinyl) méthyl]-1*H*-indole-5-carbonitrile, chlorhydrate**

Le produit attendu est obtenu selon le procédé décrit dans l'exemple 10, en remplaçant l'anhydride phtalique par le 1*H*-furo[3,4-*c*]carbazole-1,3(6*H*)-dione.

*Point de fusion :* 210-215°C

| *Microanalyse élémentaire* : | | | | |
|---|---|---|---|---|
| | C | H | N | Cl |
| % calculé | 69.20 | 5.25 | 13.02 | 6.59 |
| % trouvé | 68.70 | 5.21 | 12.65 | 6.87 |

**EXEMPLE 14 : 3-({1-[2-(3,5-Diméthyl-2,6-dioxo-3,6-dihydro-1(2*H*)-pyrimidinyl) éthyl]-3-pyrrolidinyl}-méthyl)-1*H*-indole-5-carbonitrile, chlorhydrate**

Le produit attendu est obtenu selon le procédé décrit dans l'exemple 2 en remplaçant la 3-(2-chloroéthyl)-2,4-(1*H*,3*H*)-quinazolinedione par la 3-(2-chloroéthyl)-1,5-diméthyl-2,4-(1*H*,3*H*)-pyrimidinedione.

| *Microanalyse élémentaire :* | | | | |
|---|---|---|---|---|
| | C | H | N | Cl |
| % calculé | 61.75 | 6.12 | 16.37 | 8.28 |
| % trouvé | 61.31 | 6.54 | 15.94 | 8.08 |

De la même façon, les composés suivants sont obtenus :

**<u>EXEMPLE 15 :</u> 3-[(1-{2-[2,4-Dioxo-1,4-dihydro-3(2*H*)-quinazolinyl]éthyl}-3-pyrrolidinyl)méthyl]-1*H*-indole-6-carbonitrile, chlorhydrate**

**<u>EXEMPLE 16 :</u> 3-({1-[2-(7-Méthyl-5-oxo-(5*H*)-[1,3]thiazolo[3,2-*a*]pyrimidin-6-yl) éthyl]-3-pyrrolidinyl}méthyl)-1*H*-indole-6-carbonitrile, chlorhydrate**

**<u>EXEMPLE 17 :</u> 3-({1-[2-(6-Chloro-2-oxo-2,3-dihydro-1*H*-indol-5-yl)éthyl]-3-pyrrolidinyl}méthyl)-1*H*-indole-6-carbonitrile, chlorhydrate**

**<u>EXEMPLE 18 :</u> 3-({1-[2-(1,3-Dioxo-1,3-dihydro-2*H*-isoindol-2-yl)éthyl]-3-pyrrolidinyl}méthyl)-1*H*-indole-6-carbonitrile, chlorhydrate**

**<u>EXEMPLE 19 :</u> 3-[(1-{2-[6-Méthyl-1,3-dioxo-3,6-dihydropyrrolo[3,4-*c*]carbazol-2(1*H*)-yl]éthyl}-3-pyrrolidinyl) méthyl]-1*H*-indole-6-carbonitrile, chlorhydrate**

**<u>EXEMPLE 20 :</u> 3-({1-[2-(3,5-Diméthyl-2,6-dioxo-3,6-dihydro-1(2*H*)-pyrimidinyl) éthyl]-3-pyrrolidinyl}-méthyl)-1*H*-indole-6-carbonitrile, chlorhydrate**

**<u>ETUDE PHARMACOLOGIQUE</u>**

**<u>EXEMPLE A:</u> Détermination de l'affinité pour les sites de recapture de la sérotonine chez le rat**

L'affinité des composés de l'invention a été déterminée par des expériences de compétition avec le [$^3$H]-paroxetine. Les membranes sont préparées à partir de cortex frontal de rat et incubées en triple avec 0.25 nM de [$^3$H]-paroxetine et le ligand froid dans un volume final de 0.4 ml, pendant 2 heures à 25°C. Le tampon d'incubation contient 50mM de TRIS-HCI (pH 7.4), 120 mM de NaCl et 5 mM de KCI. La fixation non-spécifique est déterminée avec 10 μM de citalopram. A la fin de l'incubation, le milieu est filtré au travers de filtres et lavés trois fois avec 5 ml de tampon refroidit. La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation ($K_i$) par l'intermédiaire de l'équation de Cheng-Prusoff :

$$K_1 = IC_{50} / \{(L / K_d) -1\}$$

dans laquelle L est la concentration de [$^3$H]-paroxetine et $K_d$ est la constante de dissociation (0.13 nM).

Il apparaît que les composés de l'invention possèdent une très haute affinité pour les sites de recapture de la sérotonine.

A titre d'exemple, l'affinité du composé de l'exemple 6 est de $4.10^{-10}$ M.

**<u>EXEMPLE B :</u> Test d'enfouissement des billes chez la souris**

Le test permet d'évaluer la capacité d'agents pharmacologiques à inhiber le comportement spontané d'enfouissement de billes chez la souris, cette inhibition étant prédictive d'actions antidépressive et/ou anti-impulsive.

Des souris mâles de souche NMRI pesant 20-25 g le jour de l'expérience, sont placées individuellement dans des boîtes en Macrolon (30x18x19 cm) contenant 5 cm de sciure et recouvertes par une plaque en plexiglas perforée. Vingt-quatre billes en verre "oeil de chat" sont réparties régulièrement sur la sciure à la périphérie de la boîte. Au terme de 30 minutes d'exploration libre, les animaux sont retirés de la boîte et le nombre de billes enfouies est comptabilisé.

*<u>Résultats :</u>* Il apparaît que les composés de l'invention inhibent le comportement spontané d'enfouissement de billes chez la souris. A titre d'exemple, les composés des exemples 6 et 7 possèdent des doses efficaces 50 ($ED_{50}$) de 3,9 mg/kg et 1.5 mg/kg respectivement.

**<u>EXEMPLE C :</u> Composition pharmaceutique**

| Formule de préparation pour 1000 comprimés dosés à 10 mg : | |
|---|---|
| Composé de l'exemple 6 | 10 g |

(suite)

| Formule de préparation pour 1000 comprimés dosés à 10 mg : | |
|---|---|
| Hydroxypropylcellulose | 2 g |
| Amidon de blé | 10 g |
| Lactose | 100 g |
| Stéarate de magnésium | 3 g |
| Talc | 3 g |

## Revendications

**1.** Composés de formule (I):

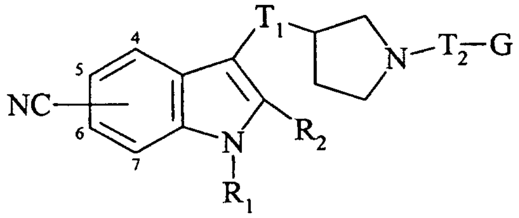

(I)

dans laquelle :

- $R_1$ et $R_2$ représentent indépendamment un atome d'hydrogène ou un groupement alkyle ($C_1$-$C_6$) linéaire ou ramifié,
- $T_1$ et $T_2$ représentent indépendamment un groupement alkylène ($C_1$-$C_6$) linéaire ou ramifié,
- G représente un groupement hétérocyclique de formule (α) ou (β) :

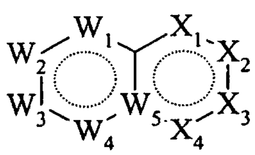

(ou)

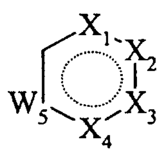

(α) (β)

dans lesquelles :
$W_1$ à $W_5$ et $X_1$ à $X_4$ sont choisis de façon à former un groupement chimiquement stable, et sont définis de la manière suivante :

- $W_1$, $W_2$ et $W_3$ représentent indépendamment un atome d'azote, un groupement $CR_5$, $NR_4$ ou CO,
- $W_4$ représente un atome d'azote, un groupement $CR_3$, $NR_4$ ou CO,
- $W_5$ représente un atome de carbone ou un atome d'azote,
- $X_1$ représente une liaison, un atome d'azote, ou un groupement $CR_3$ ou $NR_4$,
- $X_2$ à $X_4$ représentent indépendamment un groupement $CR_3$, $NR_4$, CO, $SR_4$ ou $SO_2$ ou bien un atome d'oxygène, de soufre ou d'azote,
- $R_3$ représente un atome d'hydrogène, d'halogène, un groupement alkyle ($C_1$-$C_6$) linéaire ou ramifié, hydroxy, perhalogénoalkyle ($C_1$-$C_6$) linéaire ou ramifié, nitro, ou amino (éventuellement substitué par un ou deux groupements alkyle ($C_1$-$C_6$) linéaire ou ramifié, ou benzyle),
- $R_4$ représente un atome d'hydrogène ou un groupement alkyle ($C_1$-$C_6$) linéaire ou ramifié, aryle éventuellement

substitué, arylalkyle éventuellement substitué,

- $R_5$ représente un groupement $R_3$ ou bien deux groupements $R_5$ adjacents forment avec les atomes de carbone qui les portent un groupement mono ou bicyclique saturé, partiellement insaturé ou insaturé, contenant éventuellement de 1 à 5 hétéroatomes choisis parmi azote, oxygène et soufre, ledit groupement étant éventuellement substitué par un ou plusieurs groupements $R_3$ ou oxo,

étant entendu que dans les formules (α) et (β) au moins un hétéroatome est présent, que les pointillés signifient que ces groupements peuvent comporter une insaturation ou plusieurs insaturations conjuguées ou non, et que en cas d'absence d'insaturation les valences restantes sont occupées par des atomes d'hydrogène, les groupements (α) et (β) étant reliés à $T_2$ par l'un quelconque de leur sommet,
leurs énantiomères, diastéréoisomères, ainsi que leurs sels d'addition à un acide ou à une base pharmaceutiquement acceptable,
étant entendu que :

- par groupement aryle on entend un groupement phényle ou naphtyle,
- l'expression éventuellement substituée affectée aux groupements aryle ou arylalkyle signifie que ces groupements sont éventuellement substitués par un ou plusieurs groupes choisis parmi un atome d'halogène, un groupement alkyle ($C_1$-$C_6$) linéaire ou ramifié, hydroxy, perhalogénoalkyle ($C_1$-$C_6$) linéaire ou ramifié, nitro, ou amino (éventuellement substitué par un ou deux groupements alkyle ($C_1$-$C_6$) linéaire ou ramifié, ou benzyle).

**2.** Composés de formule (I) selon la revendication 1 pour lesquels le groupement cyano est rattaché en position 5 du noyau indole, leurs énantiomères, diastéréoisomères ainsi que leurs sels d'addition à un acide ou à une base pharmaceutiquement acceptable.

**3.** Composés de formule (I) selon la revendication 1 pour lesquels le groupement cyano est rattaché en position 6 du noyau indole, leurs énantiomères, diastéréoisomères ainsi que leurs sels d'addition à un acide ou à une base pharmaceutiquement acceptable.

**4.** Composés de formule (I) selon la revendication 1 pour lesquels $R_1$ et $R_2$ représentent chacun un atome d'hydrogène, leurs énantiomères, diastéréoisomères ainsi que leurs sels d'addition à un acide ou à une base pharmaceutiquement acceptable.

**5.** Composés de formule (I) selon la revendication 1 pour lesquels $T_1$ représente un groupement méthylène, leurs énantiomères, diastéréoisomères ainsi que leurs sels d'addition à un acide ou à une base pharmaceutiquement acceptable.

**6.** Composés de formule (I) selon la revendication 1, pour lesquels G est choisi parmi les groupements :
2-furyle ; 2,4-dioxo-1,4-dihydro-3(2*H*)-quinazolinyl ; 3-oxo-[1,2,4]triazolo[4,3-a] pyridin-2(3*H*)-yl ; 1-oxo-2(1*H*)-phtalazinyl ; 7-méthyl-5-oxo-5*H*-[1,3]thiazolo[3,2-a] pyrimidin-6-yl ; 6-chloro-2-oxo-2,3-dihydro-1*H*-indol-5-yl ; 2-oxo-2,3-dihydro-1*H*-indol-5-yl ; 2-oxo-1,2,3,4-tétrahydro-6-quinolinyl ; 3-benzyl-5-méthyl-2,6-dioxo-3,6-dihydro-1(2*H*)-pyrimidinyl ; 1,3-dioxo-1,3-dihydro-2*H*-isoindol-2-yl ; 1,1,3-trioxo-1,3-dihydro-2*H*-1,2-benzisothiazol-2-yl ; 1,3-dioxo-3,6-dihydropyrrolo[3,4-c] carbazol-2(1*H*)-yl ; 1,3-dioxo-1,3-dihydro-2*H*-benzo[c]isoindol-2-yl ; 3,5-diméthyl-2,6-dioxo-3,6-dihydro-1(2*H*)-pyrimidinyl,
leurs énantiomères, diastéréoisomères ainsi que leurs sels d'addition à un acide ou à une base pharmaceutiquement acceptable.

**7.** Composés de formule (I) selon la revendication 1 pour lesquels $R_1$ et $R_2$ représentent chacun un atome d'hydrogène, $T_1$ représente un groupement méthylène, $T_2$ représente un groupement alkylène et G est choisi parmi les groupements 2-furyle ; 2,4-dioxo-1,4-dihydro-3(2*H*)-quinazolinyl ; 3-oxo-[1,2,4]triazolo[4,3-a]pyridin-2(3*H*)-yl ; 1-oxo-2(1*H*)-phtalazinyl ; 7-méthyl-5-oxo-5*H*-[1,3]thiazolo[3,2-a]pyrimidin-6-yl ; 6-chloro-2-oxo-2,3-dihydro-1*H*-indol-5-yl ; 2-oxo-2,3-dihydro-1*H*-indol-5-yl ; 2-oxo-1,2,3,4-tétrahydro-6-quinolinyl ; 3-benzyl-5-méthyl-2,6-dioxo-3,6-dihydro-1(2*H*)-pyrimidinyl ; 1,3-dioxo-1,3-dihydro-2*H*-isoindol-2-yl ; 1,1,3-trioxo-1,3-dihydro-2*H*-1,2-benzisothiazol-2-yl ; 1,3-dioxo-3,6-dihydropyrrolo[3,4-c] carbazol-2(1*H*)-yl ; 1,3-dioxo-1,3-dihydro-2*H*-benzo[c]isoindol-2-yl ; 3,5-diméthyl-2,6-dioxo-3,6-dihydro-1(2*H*)-pyrimidinyl,
leurs énantiomères, diastéréoisomères ainsi que leurs sels d'addition à un acide ou à une base pharmaceutiquement acceptable.

**8.** Composés de formule (I) selon la revendication 7 pour lesquels le groupement cyano est rattaché en position 5 du noyau indole, leurs énantiomères, diastéréoisomères ainsi que leurs sels d'addition à un acide ou à une base pharmaceutiquement acceptable.

**9.** Composés de formule (I) selon la revendication 7 pour lesquels le groupement cyano est rattaché en position 6 du noyau indole, leurs énantiomères, diastéréoisomères ainsi que leurs sels d'addition à un acide ou à une base pharmaceutiquement acceptable.

**10.** Composé de formule (I) selon la revendication 1 qui est le :

- 3-({1-[2-(7-Méthyl-5-oxo-5*H*)-[1,3]thiazolo[3,2-a]pyrimidin-6-yl)éthyl]-3-pyrrolidinyl}méthyl)-1*H*-indole-5-carbonitrile, chlorhydrate.

**11.** Composé de formule (I) selon la revendication 1 qui est le :

- 3-({1-[2-(6-Chloro-2-oxo-2,3-dihydro-1*H*-indol-5-yl)éthyl]-3-pyrrolidinyl}méthyl)-1*H*-indole-5-carbonitrile, chlorhydrate.

**12.** Procédé de préparation des composés de formule (I) selon la revendication 1 caractérisé en en ce que l'on utilise comme produit de départ un composé de formule (II) :

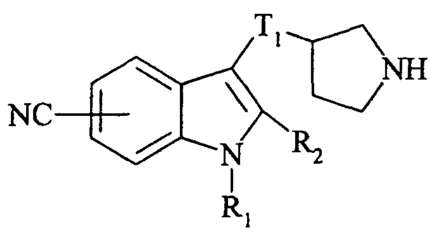

(II)

dans laquelle $R_1$, $R_2$ et $T_1$ sont tels que définis dans la formule (I),
qui est soumis :

↳ soit à l'action, en milieu réducteur d'un aldéhyde de formule (III) :

$$OHC\text{-}T'_2\text{-}G \quad \text{(III)}$$

dans laquelle G est tel que défini dans la formule (I), et $T'_2$ représente une liaison ou un groupement alkylène ($C_1$-$C_5$) linéaire ou ramifié,

↳ soit à l'action, en milieu basique d'un composé halogéné de formule (IV) :

$$Hal\text{-}T_2\text{-}G \quad \text{(IV)}$$

dans laquelle $T_2$ est tel que défini dans la formule (I) et Hal représente un atome d'halogène,

pour conduire à un composé de formule (I),
ou bien qui est soumis à l'action, en milieu basique d'un dérivé de formule (V) :

$$Hal\text{-}T_2\text{-}NH\text{-}P' \quad \text{(V)}$$

dans laquelle T2 est tel que défini dans la formule (I), Hal représente un atome d'halogène, et P' un groupement protecteur de l'amine,
pour conduire à un composé de formule (VI),

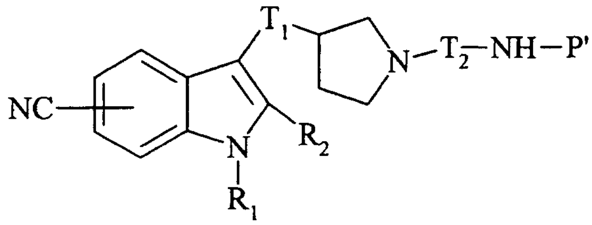

(VI)

dans laquelle $R_1$, $R_2$, $T_1$ $T_2$, P' sont tels que définis précédemment,
composé (VI) qui, après déprotection de l'amine primaire, est condensé sur un groupement cyclique, précurseur du groupement G défini dans la formule (I), pour conduire au composé de formule (I/a) :

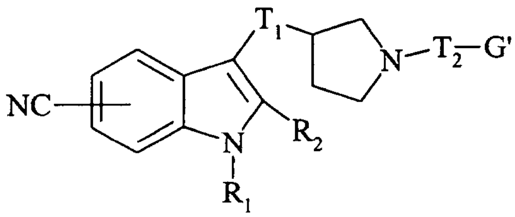

(I/a)

cas particulier des composés de formule (I) pour lequel $R_1$, $R_2$, $T_1$ $T_2$ sont tels que définis précédemment, et G' représente un groupement tel que défini pour G dans la formule (I), étant entendu que G' est rattaché à $T_2$ par un atome d'azote,
composés de formule (I/a) et (I),

- qui peuvent être, le cas échéant, purifiés selon une technique classique de purification,
- dont on sépare, le cas échéant, les isomères selon une technique classique de séparation,
- que l'on transforme, si on le souhaite, en leurs sels d'addition à un acide ou à une base pharmaceutiquement acceptable.

**13.** Compositions pharmaceutiques contenant comme principe actif au moins un composé selon l'une quelconque des revendications 1 à 11, seul ou en combinaison avec un ou plusieurs excipients ou véhicules inertes, non toxiques, pharmaceutiquement acceptables.

**14.** Compositions pharmaceutiques selon la revendication 13 contenant au moins un principe actif selon l'une quelconque des revendications 1 à 11 utiles pour la fabrication de médicaments utiles comme inhibiteurs de recapture de la sérotonine, dans le traitement de la dépression, des troubles obsessionnels compulsifs, des phobies, des troubles impulsifs de l'abus de drogue, de la boulimie nerveuse et de l'anxiété.

## Claims

**1.** Compounds of formula (I) :

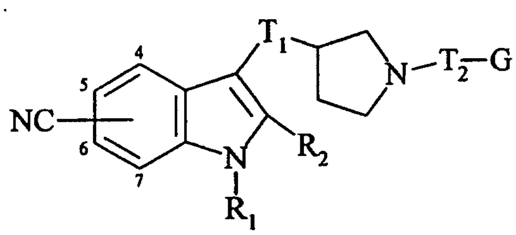

(I)

wherein :

- $R_1$ and $R_2$ each independently of the other represents a hydrogen atom or a linear or branched ($C_1$-$C_6$)alkyl group,

- $T_1$ and $T_2$ each independently of the other represents a linear or branched ($C_1$-$C_6$)-alkylene group,

- G represents a heterocyclic group of formula (α) or (β) :

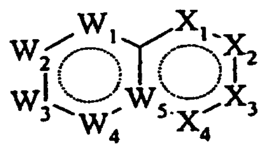

(or)

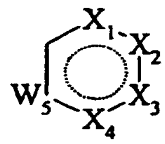

(α) (β)

wherein:

$W_1$ to $W_5$ and $X_1$ to $X_4$ are so selected as to form a chemically stable group and are defined as follows:

- $W_1$, $W_2$ and $W_3$ each independently of the others represents a nitrogen atom or a group $CR_5$, $NR_4$ or CO,
- $W_4$ represents a nitrogen atom or a group $CR_3$, $NR_4$ or CO,
- $W_5$ represents a carbon atom or a nitrogen atom,
- $X_1$ represents a bond, a nitrogen atom or a group $CR_3$ or $NR_4$,
- $X_2$ to $X_4$ each independently of the others represents a group $CR_3$, $NR_4$, CO, $SR_4$ or $SO_2$ or an oxygen, sulphur or nitrogen atom,
- $R_3$ represents a hydrogen or halogen atom or a linear or branched ($C_1$-$C_6$)alkyl group, hydroxy group, linear or branched ($C_1$-$C_6$)perhaloalkyl group, nitro group, or amino group (optionally substituted by one or two groups of linear or branched ($C_1$-$C_6$)alkyl, or benzyl),
- $R_4$ represents a hydrogen atom or a linear or branched ($C_1$-$C_6$)alkyl group, optionally substituted aryl group or optionally substituted arylalkyl group,
- $R_5$ represents a group $R_3$, or two adjacent groups $R_5$ form, together with the carbon atoms carrying them, a saturated, partially unsaturated or unsaturated mono- or bicyclic group optionally containing from 1 to 5 hetero atoms selected from nitrogen, oxygen and sulphur, the said group being optionally substituted by one or more groups $R_3$ or oxo,

it being understood that, in formulae (α) and (β), at least one hetero atom is present, the dotted lines indicate that the groups in question may contain an unsaturated bond or a plurality of conjugated or unconjugated unsaturated bonds and that, if there is no unsaturated bond, the remaining valences are occupied by hydrogen atoms, the groups (α) and (β) being linked to $T_2$ by any one of their ring junctions,

their enantiomers and diastereoisomers, and addition salts thereof with a pharmaceutically acceptable acid or base,

- an aryl group being understood to mean a phenyl or naphthyl group,
- the expression "optionally substituted" applied to aryl or arylalkyl groups being understood to mean that the groups in question are optionally substituted by one or more groups selected from halogen atoms, linear or branched ($C_1$-$C_6$)alkyl groups, hydroxy groups, linear or branched ($C_1$-$C_6$)perhaloalkyl groups, nitro groups, and amino groups (optionally substituted by one or two groups of linear or branched ($C_1$-$C_6$) alkyl, or benzyl).

**2.** Compounds of formula (I) according to claim 1, wherein the cyano group is attached in the 5-position of the indole ring system, their enantiomers and diastereoisomers, and addition salts thereof with a pharmaceutically acceptable acid or base.

**3.** Compounds of formula (I) according to claim 1, wherein the cyano group is attached in the 6-position of the indole ring system, their enantiomers and diastereoisomers, and addition salts thereof with a pharmaceutically acceptable acid or base.

4. Compounds of formula (I) according to claim 1, wherein $R_1$ and $R_2$ each represents a hydrogen atom, their enantiomers and diastereoisomers, and addition salts thereof with a pharmaceutically acceptable acid or base.

5. Compounds of formula (I) according to claim 1, wherein $T_1$ represents a methylene group, their enantiomers and diastereoisomers, and addition salts thereof with a pharmaceutically acceptable acid or base.

6. Compounds of formula (I) according to claim 1, wherein G is selected from the groups:

2-furyl; 2,4-dioxo-1,4-dihydro-3(2*H*)-quinazolinyl; 3-oxo-[1,2,4]triazolo[4,3-a]-pyridin-2(3*H*)-yl; 1-oxo-2(1*H*)-phthalazinyl; 7-methyl-5-oxo-5*H*-[1,3]thiazolo-[3,2-a]pyrimidin-6-yl; 6-chloro-2-oxo-2,3-dihydro-1*H*-indol-5-yl; 2-oxo-2,3-dihydro-1*H*-indol-5-yl; 2-oxo-1,2,3,4-tetrahydro-6-quinolinyl; 3-benzyl-5-methyl-2,6-dioxo-3,6-dihydro-1(2*H*)-pyrimidinyl; 1,3-dioxo-1,3-dihydro-2*H*-isoindol-2-yl; 1,1,3-trioxo-1,3-dihydro-2*H*-1,2-benzisothiazol-2-yl; 1,3-dioxo-3,6-dihydropyrrolo-[3,4-c]carbazol-2(1*H*)-yl; 1,3-dioxo-1,3-dihydro-2*H*-benzo[c]isoindol-2-yl; 3,5-dimethyl-2,6-dioxo-3,6-dihydro-1(2*H*)-pyrimidinyl,
their enantiomers and diastereoisomers, and addition salts thereof with a pharmaceutically acceptable acid or base.

7. Compounds of formula (I) according to claim 1, wherein $R_1$ and $R_2$ each represents a hydrogen atom, $T_1$ represents a methylene group, $T_2$ represents an alkylene group and G is selected from the groups 2-furyl; 2,4-dioxo-1,4-dihydro-3(2*H*)-quinazolinyl; 3-oxo-[1,2,4]triazolo[4,3-a]pyridin-2(3*H*)-yl; 1-oxo-2(1*H*)-phthalazinyl; 7-methyl-5-oxo-5*H*-[1,3]thiazolo[3,2-a]pyrimidin-6-yl; 6-chloro-2-oxo-2,3-dihydro-1*H*-indol-5-yl; 2-oxo-2,3-dihydro-1*H*-indol-5-yl; 2-oxo-1,2,3,4-tetrahydro-6-quinolinyl; 3-benzyl-5-methyl-2,6-dioxo-3,6-dihydro-1(2*H*)-pyrimidinyl; 1,3-dioxo-1,3-dihydro-2*H*-isoindol-2-yl; 1,1,3-trioxo-1,3-dihydro-2*H*-1,2-benzisothiazol-2-yl; 1,3-dioxo-3,6-dihydropyrrolo [3,4-c]carbazol-2(1*H*)-yl; 1,3-dioxo-1,3-dihydro-2*H*-benzo[c]iso-indol-2-yl; 3,5-dimethyl-2,6-dioxo-3,6-dihydro-1 (2*H*)-pyrimidinyl,
their enantiomers and diastereoisomers, and addition salts thereof with a pharmaceutically acceptable acid or base.

8. Compounds of formula (I) according to claim 7, wherein the cyano group is attached in the 5-position of the indole ring system, their enantiomers and diastereoisomers, and addition salts thereof with a pharmaceutically acceptable acid or base.

9. Compounds of formula (I) according to claim 7, wherein the cyano group is attached in the 6-position of the indole ring system, their enantiomers and diastereoisomers, and addition salts thereof with a pharmaceutically acceptable acid or base.

10. Compound of formula (I) according to claim 1 that is:

- 3-({1-[2-(7-methyl-5-oxo-5*H*)-[1,3]thiazolo[3,2-a]pyrimidin-6-yl)ethyl]-3-pyrrolidinyl}methyl)-1*H*-indole-5-carbonitrile hydrochloride.

11. Compound of formula (I) according to claim 1 that is :

- 3-({1-[2-(6-chloro-2-oxo-2,3-dihydro-1*H*-indol-5-yl)ethyl]-3-pyrrolidinyl}-methyl)-1*H*-indole-5-carbonitrile hydrochloride.

12. Process for the preparation of compounds of formula (I) according to claim 1, **characterised in that** there is used as starting material a compound of formula (II) :

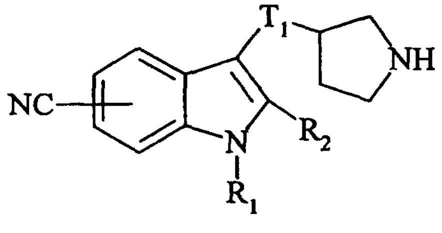

(II)

wherein $R_1$, $R_2$ and $T_1$ are as defined for formula (I), which is subjected:

- either to the action, in a reducing medium, of an aldehyde of formula (III) :

$$OHC\text{-}T'_2\text{-}G \quad (III)$$

wherein G is as defined for formula (I) and $T'_2$ represents a bond or a linear or branched ($C_1$-$C_5$)alkylene group,

- or to the action, in a basic medium, of a halogenated compound of formula (IV) :

$$Hal\text{-}T_2\text{-}G \quad (IV)$$

wherein $T_2$ is as defined for formula (I) and Hal represents a halogen atom,

to yield a compound of formula (I),
or which is subjected to the action, in a basic medium, of a compound of formula (V) :

$$Hal\text{-}T_2\text{-}NH\text{-}P' \quad (V)$$

wherein $T_2$ is as defined for formula (I), Hal represents a halogen atom and P' is a protecting group for the amine,
to yield a compound of formula (VI),

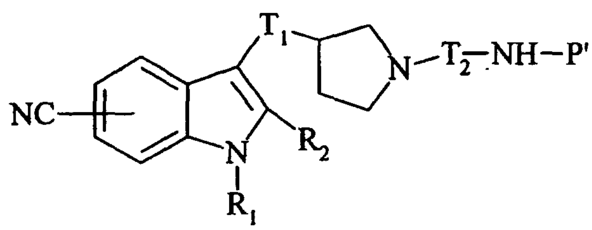

(VI)

wherein $R_1$, $R_2$, $T_1$, $T_2$ and P' are as defined hereinbefore,
which compound (VI), after deprotection of the primary amine group, is condensed with a cyclic group that is a precursor of group G defined for formula (I) to yield a compound of formula (I/a) :

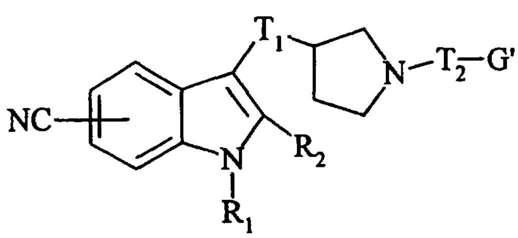

(I/a)

a particular case of the compounds of formula (I) wherein $R_1$, $R_2$, $T_1$ and $T_2$ are as defined hereinbefore and G' represents a group as defined for G in formula (I), it being understood that G' is attached to $T_2$ by a nitrogen atom, which compounds of formulae (I/a) and (I),

- may be purified, if necessary, according to a conventional purification technique,
- are separated, where appropriate, into their isomers according to a conventional separation technique,
- are converted, if desired, into addition salts thereof with a pharmaceutically acceptable acid or base.

**13.** Pharmaceutical compositions comprising as active ingredient at least one compound according to any one of

claims 1 to 11, alone or in combination with one or more inert, non-toxic pharmaceutically acceptable excipients or carriers.

**14.** Pharmaceutical compositions according to claim 13 comprising at least one active ingredient according to any one of claims 1 to 11 for use in the manufacture of medicaments for use as serotonin-reuptake inhibitors in the treatment of depression, obsessive-compulsive disorders, phobias, impulsive disorders associated with drug abuse, bulimia nervosa and anxiety.

## Patentansprüche

**1.** Verbindungen der Formel (I):

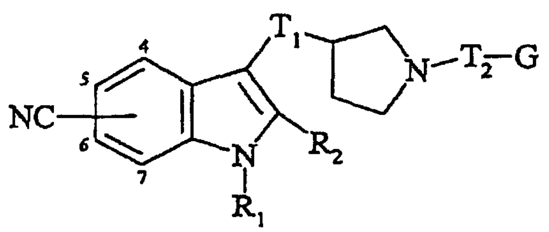

(I)

in der:

- $R_1$ und $R_2$ unabhängig voneinander ein Wasserstoffatom oder eine geradkettige oder verzweigte $(C_1\text{-}C_6)$-Alkylgruppe bedeuten,
- $T_1$ und $T_2$ unabhängig voneinander eine geradkettige oder verzweigte $(C_1\text{-}C_6)$-Alkylengruppe bedeuten,
- G eine heterocyclische Gruppe der Formel (α) oder (β) darstellt:

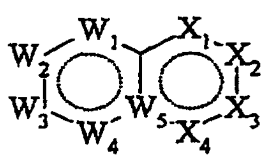

oder

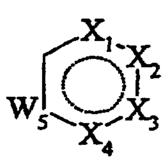

(α) (β)

in denen:

$W_1$ bis $W_5$ und $X_1$ bis $X_4$ in der Weise ausgewählt sind, daß sie eine chemisch stabile Gruppe bilden, und wie folgt definiert sind:

- $W_1$, $W_2$ und $W_3$ unabhängig voneinander ein Stickstoffatom oder eine Gruppe $CR_5$, $NR_4$, oder CO bedeuten,
- $W_4$ ein Stickstoffatom oder eine Gruppe $CR_3$, $NR_4$ oder CO bedeutet,
- $W_5$ ein Kohlenstoffatom oder ein Stickstoffatom darstellt,
- $X_1$ eine Bindung, ein Stickstoffatom oder eine Gruppe $CR_3$ oder $NR_4$ bedeutet,
- $X_2$ bis $X_4$ unabhängig voneinander eine Gruppe $CR_3$, $NR_4$, CO, $SR_4$ oder $SO_2$ oder ein Sauerstoffatom, Schwefelatom oder Stickstoffatom bedeuten,
- $R_3$ ein Wasserstoffatom, ein Halogenatom, eine geradkettige oder verzweigte $(C_1\text{-}C_6)$-Alkylgruppe, Hydroxygruppe, geradkettige oder verzweigte $(C_1\text{-}C_6)$-Perhalogenalkylgruppe, Nitrogruppe oder Aminogruppe (die gegebenenfalls durch ein oder zwei geradkettige oder verzweigte $(C_1\text{-}C_6)$-Alkylgruppe oder Benzylgruppe substituiert ist) bedeutet,
- $R_4$ ein Wasserstoffatom oder eine geradkettige oder verzweigte $(C_1\text{-}C_6)$-Alkylgruppe, gegebenenfalls substituierte Arylgruppe oder gegebenenfalls substitutierte Arylalkylgruppe darstellt,

- $R_5$ eine Gruppe $R_3$ bedeutet oder zwei benachbarte Gruppen $R_5$ mit den sie tragenden Kohlenstoffatomen eine gesättigte, teilweise ungesättigte oder ungesättigte mono- oder bicyclische Gruppe bilden, welche gegebenenfalls 1 bis 5 Heteroatome, ausgewählt aus Stickstoff, Sauerstoff und Schwefel, enthält, welche Gruppe gegebenenfalls durch ein oder zwei Gruppen $R_3$ oder Oxogruppen substituiert ist,

wobei es sich versteht, daß in den Formeln (α) und (β) mindestens ein Heteroatom vorhanden ist, die punktierten Linien bedeuten, daß diese Gruppen eine oder mehrere konjugierte oder nicht konjugierte Unsättigungen aufweisen können und daß im Fall der Abwesenheit von Unsättigung die verbleibenden Wertigkeiten durch Wasserstoffatome abgesättigt sind, wobei die Gruppen (α) und (β) über irgendeines ihrer Ringatome an $T_2$ gebunden sind, deren Enantiomere, Diastereoisomere sowie deren Additionssalze mit einer pharmazeutisch annehmbaren Säure oder Base,
wobei es sich versteht, daß:

- man unter einer Arylgruppe eine Phenyl- oder Naphthyl-Gruppe versteht,
- der Begriff gegebenenfalls substituiert unter Bezugnahme auf die Aryl- oder Arylalkyl-Gruppen bedeutet, daß diese Gruppen gegebenenfalls durch eine oder mehrere Gruppen substituiert sind, ausgewählt aus Halogenatomen, geradkettigen oder verzweigten ($C_1$-$C_6$)-Alkylgruppen, Hydroxygruppen, geradkettigen oder verzweigten ($C_1$-$C_6$)-Perhalogenalkylgruppen, Nitrogruppen oder Aminogruppen (die gegebenenfalls durch ein oder zwei geradkettige oder verzweigte ($C_1$-$C_6$)-Alkylgruppen oder Benzylgruppen substituiert sind).

**2.** Verbindungen der Formel (I) nach Anspruch 1, bei denen die Cyanogruppe in der 5-Stellung des Indolkerns gebunden ist, deren Enantiomere, Diastereoisomere sowie deren Additionssalze mit einer pharmazeutisch annehmbaren Säure oder Base.

**3.** Verbindungen der Formel (I) nach Anspruch 1, worin die Cyanogruppe in der 6-Stellung des Indolkerns gebunden ist, deren Enantiomere, Diastereoisomere sowie deren Additionssalze mit einer pharmazeutisch annehmbaren Säure oder Base.

**4.** Verbindungen der Formel (I) nach Anspruch 1, worin $R_1$ und $R_2$ jeweils ein Wasserstoffatom bedeuten, deren Enantiomere, Diastereoisomere sowie deren Additionssalze mit einer pharmazeutisch annehmbaren Säure oder Base.

**5.** Verbindungen der Formel (I) nach Anspruch 1, worin $T_1$ eine Methylengruppe bedeutet, deren Enantiomere, Diastereoisomere sowie deren Additionssalze mit einer pharmazeutisch annehmbaren Säure oder Base.

**6.** Verbindungen der Formel (I) nach Anspruch 1, worin G ausgewählt ist aus den folgenden Gruppen:

2-Furyl; 2,4-Dioxo-1,4-dihydro-3(*2H*)-chinazolinyl; 3-Oxo-[1,2,4]-triazolo-[4,3-a]pyridin-2(*3H*)-yl; 1-Oxo-2(*1H*)-phthalazinyl; 7-Methyl-5-oxo-5*H*-[1,3]-thiazolo[3,2-a]pyrimidin-6-yl; 6-Chlor-2-oxo-2,3-dihydro-1*H*-indol-5-yl;
2-Oxo-2,3-dihydro-1*H*-indol-5-yl; 2-Oxo-1,2,3,4-tetrahydro-6-chinolinyl;
3-Benzyl-5-methyl-2,6-dioxo-3,6-dihydro-1(*2H*)-pyrimidinyl; 1,3-Dioxo-1,3-dihydro-*2H*-isoindol-2-yl; 1,1,3-Trioxo-1,3-dihydro-*2H*-1,2-benzisothiazol-2-yl; 1,3-Dioxo-3,6-dihydropyrrolo[3,4-c]carbazol-2(*1H*)-yl; 1,3-Dioxo-1,3-dihydro-*2H*-benzo[c]-isoindol-2-yl; 3,5-Dimethyl-2,6-dioxo-3,6-dihydro-1(*2H*)-pyrimidinyl,

deren Enantiomere, Diastereoisomere sowie deren Additionsalze mit einer pharmazeutisch annehmbaren Säure oder Base.

**7.** Verbindungen der Formel (I) nach Anspruch 1, worin $R_1$ und $R_2$ jeweils ein Wasserstoffatom bedeuten, $T_1$ eine Methylengruppe darstellt, $T_2$ eine Alkylengruppe bedeutet und G ausgewählt ist aus den folgenden Gruppen: 2-Furyl;
2,4-Dioxo-1,4-dihydro-3(2*H*)-chinazolinyl; 3-Oxo-[1,2,4]triazolo[4,3-a]-pyridin-2(3*H*)-yl; 1-Oxo-2(1*H*)-phthalazinyl; 7-Methyl-5-oxo-5*H*-[1,3]thiazolo[3,2-a]-pyrimidin-6-yl; 6-Chlor-2-oxo-2.3-dihydro-*1H*-indol-5-yl; 2-Oxo-2,3-dihydro-*1H*-indol-5-yl; 2-Oxo-1,2,3,4-tetrahydro-6-chinolinyl; 3-Benzyl-5-methyl-2,6-dioxo-3,6-dihydro-1(*2H*)-pyrimidinyl; 1,3-Dioxo-1,3-dihydro-*2H*-isoindol-2-yl; 1,1,3-Trioxo-1,3-dihydro-2*H*-1,2-benzisothiazol-2-yl; 1,3-Dioxo-3,6-dihydropyrrolo[3,4-c]carbazol-2(1*H*)-yl; 1,3-Dioxo-1,3-dihydro-2*H*-benzo[c]-isoindol-2-yl; 3,5-Dimethyl-2,6-dioxo-3,6-dihydro-1(2*H*)-pyrimidinyl,
deren Enantiomere, Diastereoisomere sowie deren Additionsalze mit einer pharmazeutisch annehmbaren Säure

oder Base.

**8.** Verbindungen der Formel (I) nach Anspruch 7, worin die Cyanogruppe in der 5-Stellung des Indolkerns gebunden ist, deren Enantiomere, Diastereoisomere sowie der Additionssalze mit einer pharmazeutisch annehmbaren Säure oder Base.

**9.** Verbindungen der Formel (I) nach Anspruch 7, worin die Cyanogruppe in der 6-Stellung des Indolkerns gebunden ist, deren Enantiomere, Diastereoisomere sowie der Additionssalze mit einer pharmazeutisch annehmbaren Säure oder Base.

**10.** Verbindung der Formel (I) nach Anspruch 1, nämlich:

- 3-({1-(2-(7-Methyl-5-oxo-*5H*)-[1,3]-thiazolo[3,2-a]pyrimidin-6-yl)-ethyl]-3-pyrrolidinyl}-methyl)-*1H*-indol-5-carbonitril, Hydrochlorid.

**11.** Verbindung der Formel (I) nach Anspruch 1, nämlich:

- 3-({1-[2-(6-Chlor-2-oxo-2,3-dihydro-*1H*-indol-5-yl)-ethyl]-3-pyrrolidinyl}methyl)-*1H*-indol-5-carbonitril, Hydrochlorid.

**12.** Verfahren zur Herstellung der Verbindungen der Formel (I) nach Anspruch 1, **dadurch gekennzeichnet, daß** man als Ausgangsprodukt eine Verbindung der Formel (II) verwendet:

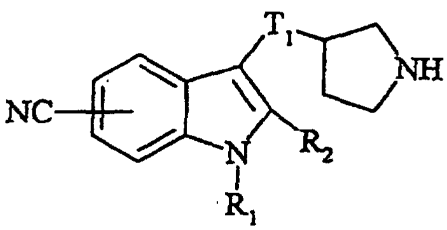

(II)

in der $R_1$, $R_2$ und $T_1$ die bezüglich der Formel (I) angegebenen Bedeutungen besitzen, welche man

↳ entweder der Einwirkung eines Aldehyds der Formel (III):

$$OHC\text{-}T'_2\text{-}G \quad \text{(III)}$$

in der G die bezüglich der Formel (I) angegebenen Bedeutungen besitzt und $T'_2$ eine Bindung oder eine geradkettige oder verzweigte ($C_1$-$C_5$)-Alkylengruppe darstellt, in reduzierendem Medium unterwirft,

↳ oder der Einwirkung einer Halogenverbindung der Formel (IV):

$$Hal\text{-}T_2\text{-}G \quad \text{(IV)}$$

in der $T_2$ die bezüglich der Formel (I) angegebenen Bedeutungen besitzt und Hal ein Halogenatom darstellt, in basischem Medium unterwirft,

zur Bildung einer Verbindung der Formel (I)),
oder der Einwirkung eines Derivats der Formel (V):

$$Hal\text{-}T_2\text{-}NH\text{-}P' \quad \text{(V)}$$

in der $T_2$ die bezüglich der Formel (I) angegebenen Bedeutungen besitzt, Hal ein Halogenatom darstellt und P' eine Schutzgruppe für das Amin darstellt, in basischem Medium unterwirft,
zur Bildung einer Verbindung der Formel (VI):

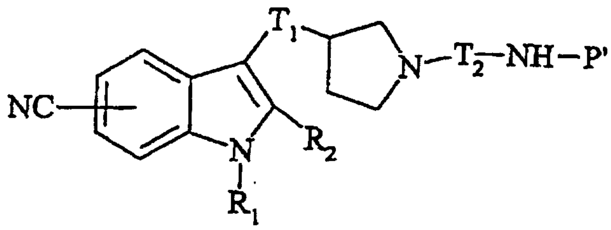

(VI)

in der $R_1$, $R_2$, $T_1$, $T_2$ und P' die oben angegebenen Bedeutungen besitzen,
welche Verbindung (VI) man nach der Abspaltung der Schutzgruppe des primären Amins mit einer cyclischen Gruppe, die einen Vorläufer der in der Formel (I) definierten Gruppe G darstellt, kondensiert, zur Bildung der Verbindung der Formel (I/a):

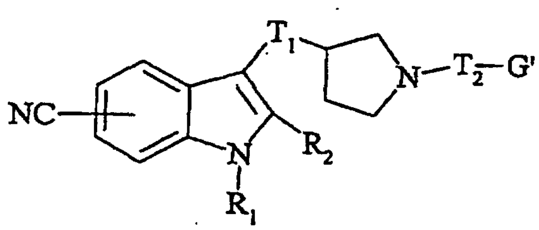

(I/a)

einem Sonderfall der Verbindungen der Formel (I), in der $R_1$, $R_2$, $T_1$ und $T_2$ die oben angegebenen Bedeutungen besitzen und G' eine Gruppe ist, wie sie für G bezüglich der Formel (I) definiert worden ist, wobei es sich versteht, daß G' über ein Stickstoffatom an $T_2$ gebunden ist,
welche Verbindungen der Formeln (I/a) und (I),

- gegebenenfalls mit Hilfe einer klassischen Reinigungsmethode gereinigt werden können,
- man gegegebenenfalls mit Hilfe einer klassischen Trennmethode in ihre Isomeren auftrennt, und
- man gewünschtenfalls in ihre Additionssalze mit einer pharmazeutisch annehmbaren Säure oder Base überführt.

**13.** Pharmazeutische Zubereitungen enthaltend als Wirkstoff mindestens eine Verbindung nach einem der Anspüche 1 bis 11 allein oder in Kombination mit einem oder mehreren inerten, nicht-toxischen und pharmazeutisch annehmbaren Trägermaterialien oder Bindemitteln.

**14.** Pharmazeutische Zubereitungen nach Anspruch 13 enthaltend mindestens einen Wirkstoff nach einem der Ansprüche 1 bis 11 für die Herstellung von Arzneimitteln, die nützlich sind als Inhibitoren der Wiederaufnahme von Serotonin, für die Behandlung von Depressionen, von krampfartigen Besessenheitsstörungen, von Phobien, von impulsiven Störungen des Drogenmißbrauchs, der nervösen Bulimie und der Angst.